# EUROPÄISCHE PATENTANMELDUNG

(11) **EP 3 361 401 A1**
(43) Veröffentlichungstag der Anmeldung: **15.08.2018**
(21) Anmeldenummer: 17155589.9
(22) Anmeldetag: 10.02.2017
(51) Int. Cl.: G06F 19/00, A61H 33/06, A61N 5/06, A61H 33/00, A61B 5/00

(54) **VERFAHREN FÜR DIE NUTZUNG EINER WÄRMEKABINE**

(71) Anmelder: corso sauna manufaktur gmbh, 49565 Bramsche (DE)
(72) Erfinder: Lingens, Benedictus, 49594 Alfhausen (DE); Lingens, Max, 49594 Alfhausen (DE)
(74) Vertreter: Pelster, Arno

(57) **Zusammenfassung**

Die Erfindung betrifft ein Verfahren für die Nutzung einer Wärmekabine (12), mit dem Schritten: Erfassen von Vitalparametern (38) einer Person (14), insbesondere während der Nutzung der Wärmekabine (12), Speichern der erfassten Vitalparameter (38) in einer Datenbank (56) und Bereitstellen der Datenbank (56) zum Auswerten der gespeicherten Vitalparameter (38).

## Beschreibung

Die Erfindung betrifft ein Verfahren für die Nutzung einer Wärmekabine mit dem Schritt: Erfassen von Vitalparametern einer Person, insbesondere während der Nutzung der Wärmekabine.

Die Erfindung betrifft außerdem eine Wärmekabineneinrichtung mit einer Wärmekabine und einem oder mehreren Sensoren, welche jeweils dazu eingerichtet sind, Vitalparameter einer Person, insbesondere während der Nutzung der Wärmekabine, zu erfassen.

Wärmekabinen, wie beispielsweise Saunen oder Infrarotkabinen, erfreuen sich aufgrund des steigenden Gesundheitsbewusstseins zunehmender Beliebtheit. Insbesondere im Wellness- und Sportbereich werden Wärmekabinen vermehrt zum Entspannen und zur Steigerung des Wohlbefindens in Anspruch genommen.

Die Nutzung von Wärmekabinen kann dabei in öffentlichen Einrichtungen, wie Schwimmbädern, Fitnessstudios, Hotels oder Saunaanstalten erfolgen. Darüber hinaus werden entsprechende Wärmekabinen auch in einer beträchtlichen Anzahl von Privathaushalten genutzt.

Es hat sich gezeigt, dass die Wirkung von Wärmekabinen auf die körperliche Verfassung und das Wohlbefinden stark von dem Nutzungsverhalten der entsprechenden Personen abhängt.

Damit der gewünschte Effekt bei der die Wärmekabine nutzenden Person eintreten kann, bedarf es regelmäßig eines individuell angepassten Nutzungsverhaltens der Wärmekabine. Die jeweils für unterschiedliche Personen empfehlenswerten Nutzungsverhalten können dabei erheblich voneinander abweichen. Beispielsweise kann die zu empfehlende Nutzungsdauer, Kabinentemperatur und/oder Kabinenfeuchtigkeit in Bezug auf unterschiedliche Personen stark variieren.

Das Generieren eines Vorschlags für ein individuelles Nutzungsverhalten einer Wärmekabine ist zurzeit nur mit einem hohen zeitlichen Aufwand möglich und verursacht darüber hinaus erhebliche Kosten. Aus diesem Grund werden Wärmekabinen häufig unter Befolgung von einigen wenigen Grundregeln genutzt, ohne dass das Nutzungsverhalten an personenspezifische Parameter angepasst ist. Dies führt letztendlich dazu, dass die Nutzung von Wärmekabinen regelmäßig keine optimale Wirkung entfaltet.

Die der Erfindung zugrundeliegende Aufgabe ist es somit Personen die Möglichkeit zu geben, durch die Umsetzung eines individuellen Nutzungsverhaltens die Wirkung der Wärmekabinennutzung zu verbessern.

Die Aufgabe wird gelöst durch ein Verfahren der eingangs genannten Art, wobei die erfassten Vitalparameter in einer Datenbank gespeichert und die Datenbank zum Auswerten der gespeicherten Vitalparameter bereitgestellt wird.

Die Erfindung macht sich die Erkenntnis zunutze, dass es aufgezeichnete Vitalparameter erlauben, ein empfehlenswertes individuelles Nutzungsverhalten für die Nutzung einer Wärmekabine, wie einer Sauna oder einer Infrarotkabine, abzuleiten. Die Vitalparameter können dabei vor, während und/oder nach der Nutzung der Wärmekabine erfasst werden. Das Erfassen kann beispielsweise selbsttätig durch geeignete Sensoren, insbesondere während der Nutzung der Wärmekabine, und/oder manuell durch die Eingabe von personenspezifischen Informationen und/oder gruppenspezifischen Informationen erfolgen. Im Sinne der Erfindung sind unter Vitalparametern nicht nur situationsabhängig veränderliche Herz-Kreislauf-Parameter wie die Körpertemperatur, die Herzfrequenz, der Blutdruck, die Atemfrequenz oder die Bestandteile des Körperschweißes der Person zu verstehen, sondern ebenfalls Parameter wie das Alter, die Größe, das Gewicht oder das Geschlecht der Person. Die erfassbaren Bestandteile des Körperschweißes umfassen beispielsweise Glucose, Lactat, Natrium, Kalium und/oder andere Substanzen. Einige Parameter sind bevorzugt manuell zu erfassen. Insbesondere werden die erfassten Vitalparameter in der Datenbank einem Benutzerprofil zugeordnet. Es ist bevorzugt, dass das Benutzerprofil ein anonymisiertes Benutzerprofil ist. Den erfassten Vitalparametern werden in der Datenbank vorzugsweise weitere Attribute zugeordnet, wie beispielsweise das Erfassungsdatum, die Erfassungsuhrzeit und/oder der Erfassungsort. Ferner können die zur Erfassung der Vitalparameter verwendeten Geräte in der Datenbank hinterlegt werden.

In einer bevorzugten Ausführungsform des erfindungsgemäßen Verfahrens werden die in der Datenbank gespeicherten Vitalparameter zum Erzeugen einer Handlungsempfehlung für die Nutzung der Wärmekabine ausgewertet. Die erzeugte Handlungsempfehlung für die Nutzung der Wärmekabine kann eine personenbezogene Handlungsempfehlung für die Nutzung der Wärmekabine oder eine gruppenspezifische Handlungsempfehlung für die Nutzung der Wärmekabine sein. Alternativ oder zusätzlich wird die erzeugte Handlungsempfehlung für die Person oder Gruppe bereitgestellt. Die Bereitstellung erfolgt vorzugsweise mittels einer Anwendung auf einem mobilen Endgerät und/oder einer Wiedergabeeinrichtung der Wärmekabine. Insbesondere wird die Handlungsempfehlung der Person visuell angezeigt, beispielsweise über eine Anzeige oder ein Touchscreen des mobilen Endgeräts. Alternativ oder zusätzlich kann die Handlungsempfehlung der Person auch akustisch bereitgestellt werden. Das mobile Endgerät kann beispielsweise ein Tablet, ein Mobilfunkgerät, wie etwa ein Smartphone, oder ein Laptop sein. Alternativ oder zusätzlich kann der Person die Handlungsempfehlung auch über eine kabineneigene Anzeige, wie einem kabineneigenen Display oder einem kabineneigenen Touchscreen bereitgestellt werden. Die Bereitstellung der Handlungsempfehlung kann beispielsweise vor, während oder nach der Nutzung der Wärmekabine erfolgen.

Die Handlungsempfehlung kann beispielweise die Nutzungsdauer, die Kabinentemperatur und/oder die zu erreichende Körpertemperatur betreffen. Wenn die Wärmekabine als Sauna ausgebildet ist, kann die Handlungsempfehlung beispielsweise die Ofentemperatur und/oder die Luftfeuchtigkeit betreffen. Wenn die Wärmekabine als Infrarotkabine ausgebildet ist, kann die Handlungsempfehlung beispielsweise die Strahlungsintensität oder die Position von einem oder mehreren Infrarotstrahlern umfassen. Ferner kann die Handlungsempfehlung einen Positionsvorschlag innerhalb der Wärmekabine für die Person umfassen. Beispielsweise kann der Positionsvorschlag für die Person einen Vorschlag für eine Liege oder eine Liegefläche der Wärmekabine umfassen, welche von der Person während der Nutzung der Wärmekabine verwendet werden soll. Beispielsweise können die Liegen oder Liegeflächen der Wärmekabine eine Bezeichnung tragen, damit die vorgeschlagene Liege oder Liegefläche einfacher für die Person auffindbar ist. Ferner kann die Handlungsempfehlung der Person einen Wechsel auf eine andere Liege oder eine andere Liegefläche vorschlagen. Die Handlungsempfehlung kann auch vorbereitende Maßnahmen betreffen, welche die Person vor der Nutzung der Wärmekabine umsetzen soll. Alternativ oder zusätzlich kann die Handlungsempfehlung auch Maßnahmen betreffen, welche nach der Nutzung der Wärmekabine, insbesondere unmittelbar nach Beendigung der Nutzung der Wärmekabine, umgesetzt werden sollen. Handlungsempfehlungen, welche vor oder nach der Nutzung der Wärmekabine umsetzbar sind, können beispielsweise die Temperierung des Körpers betreffen. Die Handlungsempfehlung kann eine Empfehlung zur Steigerung der körperlichen Fitness durch Betreiben von Sport und/oder eine Ernährungsempfehlung betreffen, wobei die Ernährungsempfehlung beispielsweise das Zuführen von Wasser oder isotonischen Getränken und/oder den Verzicht auf schwer verdaulich Kost vor der Wärmekabinennutzung betreffen kann. Außerdem kann die Handlungsempfehlung die Empfehlung einen Arzt aufzusuchen, umfassen, beispielsweise aufgrund von erheblichen Abweichungen der erfassten Vitalparameter gegenüber Standardwerten.

Das erfindungsgemäße Verfahren wird außerdem dadurch vorteilhaft weitergebildet, dass die in der Datenbank gespeicherten Vitalparameter zum Erzeugen eines Steuerungsbefehls für die Wärmekabine ausgewertet werden und/oder der erzeugte Steuerungsbefehl einer Steuerungseinrichtung der Wärmekabine bereitgestellt wird. Das Auswerten der in der Datenbank gespeicherten Vitalparameter und/oder das Erzeugen eines Steuerungsbefehls für die Wärmekabine erfolgt dabei vorzugsweise in Echtzeit, sodass eine in Echtzeit gesteuerte Wärmekabinensteuerung umgesetzt wird. Alternativ oder zusätzlich zum Erzeugen einer Handlungsempfehlung für die Nutzung der Wärmekabine erlaubt das Erzeugen eines Steuerungsbefehls für die Wärmekabine eine selbsttätige beziehungsweise automatische Steuerung der Wärmekabine, ohne dass eine Handlung seitens der die Wärmekabine nutzenden Person notwendig ist. Durch die selbsttätige Steuerung der Wärmekabine kann eine äußerst präzise Anpassung der Betriebsparameter der Wärmekabine an die die Wärmekabine nutzende Person erfolgen. Weiterhin wird der Nutzungskomfort der Wärmekabine aufgrund des Wegfalls einer manuellen Einstellung von Betriebsparametern der Wärmekabine durch die die Wärmekabine nutzende Person erheblich gesteigert. Die Bereitstellung des Steuerungsbefehls kann beispielsweise vor, während oder nach der Nutzung der Wärmekabine erfolgen.

In einer anderen Ausführungsform des erfindungsgemäßen Verfahrens wird ein Ofen der Wärmekabine abhängig von dem bereitgestellten Steuerungsbefehl selbsttätig eingestellt. Das Einstellen des Ofens, welcher vorzugsweise als Saunaofen ausgebildet ist, umfasst insbesondere das Einstellen der Ofentemperatur und/oder der Heizleistung des Ofens. Durch das Einstellen der Ofentemperatur und/oder der Heizleistung des Ofens kann die Temperatur innerhalb der Wärmekabine angepasst werden, wobei das Anpassen der Temperatur innerhalb der Wärmekabine das Erhöhen und/oder das Verringern der Temperatur innerhalb der Wärmekabine umfassen kann. Der Ofen kann beispielsweise ein Holzofen, ein Gasofen, ein Elektroofen oder eine Kombination aus den zuvor genannten Öfen sein. Alternativ oder zusätzlich wird abhängig von dem bereitgestellten Steuerungsbefehl selbsttätig ein Aufguss ausgelöst und/oder gesteuert. Das Auslösen eines Aufgusses umfasst vorzugsweise das Begießen von heißen Steinen des Ofens mit einer Aufgussflüssigkeit. Die Aufgussflüssigkeit kann beispielsweise Wasser sein, wobei das Wasser mit unterschiedlichen Ölen, Aromen und/oder Mineralien versetzt sein kann. Alternativ oder zusätzlich werden abhängig von dem bereitgestellten Steuerungsbefehl ein oder mehrere Infrarotstrahler der Wärmekabine selbsttätig eingestellt. Das selbsttätige Einstellen des einen oder der mehreren Infrarotstrahler der Wärmekabine abhängig von dem bereitgestellten Steuerungsbefehl kann das Einstellen der Strahlungsintensität, der Position und/oder der Ausrichtung des einen oder der mehreren Infrarotstrahler umfassen. Alternativ oder zusätzlich wird abhängig von dem bereitgestellten Steuerungsbefehl ein Möbel der Wärmekabine selbsttätig eingestellt. Das Möbel kann beispielsweise eine Liege oder ein Stuhl sein, wobei das Möbel fest in der Kabine integriert oder als portables Möbel ausgebildet sein kann. Alternativ oder zusätzlich werden abhängig von dem bereitgestellten Steuerungsbefehl ein oder mehrere Lüfter der Wärmekabine selbsttätig eingestellt. Das selbsttätige Einstellen des einen oder der mehreren Lüfter der Wärmekabine abhängig von dem bereitgestellten Steuerungsbefehl kann das Einstellen der Strömungsgeschwindigkeit und/oder der Strömungsrichtung der von dem einen oder den mehreren Lüftern beschleunigten Luft, und/oder das Einstellen der Position und/oder der Ausrichtung des einen oder der mehreren Lüfter umfassen. Der eine oder die mehreren Lüfter sind vorzugsweise an dem Möbel angeordnet und/oder befestigt.

In einer besonders bevorzugten Ausführungsform des erfindungsgemäßen Verfahrens ist das Möbel der Wärmekabine als Liege ausgebildet und das selbsttätige Einstellen des Möbels der Wärmekabine abhängig von dem bereitgestellten Steuerungsbefehl umfasst das Einstellen der Position, insbesondere der Höhe, der Liegefläche der Liege und/oder das Einstellen der Neigung von einem oder mehreren Abschnitten der Liegefläche der Liege. Durch das Einstellen der Höhe der Liegefläche der Liege kann die sich auf der Liege befindliche Person in eine andere Temperaturzone verfahren werden. Darüber hinaus kann die Entfernung der Liege zu einem Ofen oder einem oder mehreren Infrarotstrahlern der Wärmekabine durch ein Einstellen der Position der Liegefläche der Liege erfolgen. Das Einstellen der Neigung von einem oder mehreren Abschnitten der Liegefläche der Liege kann beispielsweise das Ändern der Neigung der Rückenlehne der Liege und/oder das Ändern der Neigung des Bein- beziehungsweise Wadenablageabschnitts der Liege umfassen.

In einer weiteren Ausführungsform des erfindungsgemäßen Verfahrens umfasst das Erfassen von Vitalparametern der Person während der Nutzung der Wärmekabine das Erfassen der Temperatur von einer oder mehreren Körperregionen der Person während der Nutzung der Wärmekabine. Das Erfassen der Temperatur von einer oder mehreren Körperregionen der Person während der Nutzung der Wärmekabine erfolgt vorzugsweise mittels einem oder mehreren Temperatursensoren, wobei der eine oder die mehreren Temperatursensoren zur Erfassung von Wärmestrahlung ausgebildet sein können. Das Erfassen der Temperatur von einer oder mehreren Körperregionen der Person während der Nutzung der Wärmekabine kann berührungslos und/oder durch eine Berührung der entsprechenden Körperregion erfolgen. Das Erfassen von Bestandteilen innerhalb des Körperschweißes der Person erfolgt vorzugsweise über einen oder mehrere Biosensoren. Alternativ oder zusätzlich umfasst das Erfassen von Vitalparametern der Person während der Nutzung der Wärmekabine das Erfassen der Herzfrequenz der Person während der Nutzung der Wärmekabine. Die Herzfrequenz der Person kann beispielsweise mittels eines elektronischen Messgeräts in einem Brustgurt, einem Armband, einem Ohreinsatz, einem Fingeraufsatz, einer Fingerklemme und/oder einem anderen am Körper tragbaren Gerät erfolgen, wobei der Brustgurt, das Armband, der Ohreinsatz, der Fingeraufsatz, die Fingerklemme und/oder das andere am Körper tragbare Gerät auch zur Erfassung der Temperatur von einer oder mehreren Körperregionen der Person dienen kann. Außerdem kann die Herzfrequenzerfassung mittels einer Messeinrichtung an einem Möbel der Wärmekabine, beispielsweise einem Handgriff, erfolgen. Alternativ oder zusätzlich umfasst das Erfassen von Vitalparametern der Person während der Nutzung der Wärmekabine das Erfassen des Blutdrucks der Person während der Nutzung der Wärmekabine. Die Blutdruckmessung kann beispielsweise am Handgelenk, am Oberarm oder am Finger der die Wärmekabine nutzenden Person erfolgen. Alternativ oder zusätzlich umfasst das Erfassen von Vitalparametern der Person während der Nutzung der Wärmekabine das Erfassen der Atemfrequenz der Person während der Nutzung der Wärmekabine. Das Erfassen der Atemfrequenz kann beispielsweise durch das Erfassen von Brustumfangsveränderungen der die Wärmekabine nutzenden Person erfolgen, wobei die Brustumfangsveränderungen mittels eines Brustgurts oder durch optische Sensoren erfasst werden können. Das Erfassen von Vitalparametern der Person während der Nutzung der Wärmekabine kann in regelmäßigen zeitlichen Abständen, wie beispielsweise jede Sekunde oder jede 5 Sekunden, erfolgen, wobei die Erfassungsfrequenz der verschiedenen Vitalparameter unterschiedlich sein kann.

In einer bevorzugten Weiterbildung des erfindungsgemäßen Verfahrens befindet sich die Datenbank auf einem entfernt von der Wärmekabine positionierten Zentralrechner, wobei das Verfahren das Senden der erfassten Vitalparameter an den Zentralrechner, insbesondere unter Verwendung des Internets, umfasst. Das Auswerten der in der Datenbank gespeicherten Vitalparameter zum Erzeugen einer Handlungsempfehlung für die Nutzung der Wärmekabine und/oder das Auswerten der in der Datenbank gespeicherten Vitalparameter zum Erzeugen eines Steuerungsbefehls für die Wärmekabine erfolgt vorzugsweise durch eine Auswerteeinrichtung. Die Auswerteeinrichtung kann beispielsweise Bestandteil des Zentralrechners, der Steuerungseinrichtung der Wärmekabine, oder des mobilen Endgeräts sein. Wenn die Auswerteeinrichtung Bestandteil des mobilen Endgeräts ist, erfolgt das Auswerten der in der Datenbank gespeicherten Vitalparameter zum Erzeugen einer Handlungsempfehlung für die Nutzung der Wärmekabine und/oder das Auswerten der in der Datenbank gespeicherten Vitalparameter zum Erzeugen eines Steuerungsbefehls für die Wärmekabine insbesondere durch die Anwendung auf dem mobilen Endgerät. Vorzugsweise weist die Wärmekabine, insbesondere die Steuerungseinrichtung der Wärmekabine, ein Kommunikationsmodul auf, mittels welchem die erfassten Vitalparameter, beispielsweise unter Verwendung eines Netzwerks, unter Verwendung von Mobilfunk und/oder über das Internet an den Zentralrechner gesendet werden können. Außerdem ist es bevorzugt, dass die von dem einen oder den mehreren portablen elektronischen Geräten erfassten Vitalparameter an eine Empfängereinrichtung, insbesondere mittels Bluetooth oder Nahfeldkommunikation, gesendet werden, sodass die Empfängereinrichtung die erfassten Vitalparameter über ein lokales Netzwerk der Steuerungseinrichtung der Wärmekabine bereitstellen kann. Die Steuerungseinrichtung kann dann die erfassten Vitalparameter über das Internet an den Zentralrechner senden. Die erfassten Vitalparameter können von der Empfängereinrichtung alternativ oder zusätzlich auch direkt oder über das mobile Endgerät, insbesondere über das Internet, an den Zentralrechner gesendet werden. Das Speichern der erfassten Vitalparameter in der Datenbank kann das Speichern der erfassten Vitalparameter von mehreren zeitlich beabstandeten Nutzungen der Wärmekabine durch die Person umfassen. Das Verfahren kann ferner das Senden von Vitalparametern, Handlungsempfehlungen und/oder Steuerungsbefehlen von dem Zentralrechner an die Steuerungseinrichtung der Wärmekabine und/oder an das mobile Endgerät umfassen.

Die der Erfindung zugrundeliegende Aufgabe wird außerdem gelöst durch eine Wärmekabineneinrichtung der eingangs genannten Art, wobei die erfindungsgemäße Wärmekabineneinrichtung eine Datenbank und eine Auswerteeinrichtung umfasst. Die Datenbank ist dazu eingerichtet, die erfassten Vitalparameter zu speichern. Die Auswerteeinrichtung ist dazu eingerichtet, die in der Datenbank gespeicherten Vitalparameter auszuwerten. Hinsichtlich der Vorteile der erfindungsgemäßen Wärmekabineneinrichtung wird auf die Vorteile des erfindungsgemäßen Verfahrens verwiesen.

In einer bevorzugten Ausführungsform der erfindungsgemäßen Wärmekabineneinrichtung ist die Auswerteeinrichtung dazu eingerichtet, auf Basis der in der Datenbank gespeicherten Vitalparameter eine Handlungsempfehlung für die Nutzung der Wärmekabine zu erzeugen und bereitzustellen. Die Auswerteeinrichtung kann dazu eingerichtet sein, auf Basis der in der Datenbank gespeicherten Vitalparameter eine personenbezogene Handlungsempfehlung für die Nutzung der Wärmekabine und/oder eine gruppenspezifische Handlungsempfehlung für die Nutzung der Wärmekabine zu erzeugen und bereitzustellen. Vorzugsweise ist die Auswerteeinrichtung direkt und/oder indirekt, beispielsweise über das Internet, mit einem mobilen Endgerät oder einer Wiedergabeeinrichtung der Wärmekabine signalleitend verbunden, sodass die Handlungsempfehlung der Person optisch und/oder akustisch wiedergegeben werden kann. Insbesondere ist die Auswerteeinrichtung dazu eingerichtet, die Handlungsempfehlung vor oder während der Nutzung der Wärmekabine bereitzustellen.

Die Handlungsempfehlung kann beispielweise die Nutzungsdauer betreffen. Wenn die Wärmekabine als Sauna ausgebildet ist, kann die Handlungsempfehlung beispielsweise die Ofen- oder Kabinentemperatur und/oder die Luftfeuchtigkeit betreffen. Wenn die Wärmekabine als Infrarotkabine ausgebildet ist, kann die Handlungsempfehlung beispielsweise die Strahlungsintensität oder die Position von einem oder mehreren Infrarotstrahlern umfassen. Ferner kann die Handlungsempfehlung einen Positionsvorschlag innerhalb der Wärmekabine für die Person umfassen.

Die erfindungsgemäße Wärmekabineneinrichtung wird außerdem durch ein mobiles Endgerät vorteilhaft weitergebildet, wobei das mobile Endgerät dazu eingerichtet ist, der Person die Handlungsempfehlung bereitzustellen, insbesondere mittels einer auf dem mobilen Endgerät installierten Anwendung und/oder einer Wiedergabeeinrichtung der Wärmekabine. Das mobile Endgerät kann außerdem dazu eingerichtet sein, Steuerungsbefehle zu erzeugen und/oder Steuerungsbefehle an eine Steuerungseinrichtung der Wärmekabineneinrichtung zu senden. Das mobile Endgerät kann beispielsweise ein Tablet, ein Mobilfunkgerät, wie etwa ein Smartphone, oder ein Laptop sein. Vorzugsweise ist das mobile Endgerät direkt, beispielsweise über Funk, insbesondere über Bluetooth oder Nahfeldkommunikation, mit der Wärmekabine signalleitend verbunden. Alternativ oder zusätzlich kann das mobile Endgerät indirekt, beispielsweise über das Internet und/oder über Mobilfunk, mit der Wärmekabine signalleitend verbunden sein.

In einer anderen Ausführungsform der erfindungsgemäßen Wärmekabineneinrichtung ist die Auswerteeinrichtung dazu eingerichtet, auf Basis der in der Datenbank gespeicherten Vitalparameter einen Steuerungsbefehl für die Wärmekabine zu erzeugen und bereitzustellen. Somit wird eine selbsttätige Steuerung der Wärmekabine erreicht, ohne dass eine Handlung seitens der die Wärmekabine nutzenden Person notwendig ist. Dadurch, dass die Auswerteeinrichtung dazu eingerichtet ist, auf Basis der in der Datenbank gespeicherten Vitalparameter einen Steuerungsbefehl für die Wärmekabine zu erzeugen und bereitzustellen, kann eine äußerst präzise Anpassung der Betriebsparameter der Wärmekabine an die die Wärmekabine nutzende Person erfolgen. Weiterhin wird der Nutzungskomfort der Wärmekabine aufgrund des Wegfalls einer manuellen Einstellung von Betriebsparametern der Wärmekabine durch die die Wärmekabine nutzende Person erheblich gesteigert. Insbesondere ist die Auswerteeinrichtung dazu eingerichtet, den Steuerungsbefehl vor oder während der Nutzung der Wärmekabine bereitzustellen.

Bevorzugt ist darüber hinaus eine Wärmekabineneinrichtung mit einer Steuerungseinrichtung, welche dazu eingerichtet ist, abhängig von dem bereitgestellten Steuerungsbefehl einen Ofen, einen oder mehrere Infrarotstrahler, ein Möbel und/oder einen oder mehrere Lüfter der Wärmekabine selbsttätig einzustellen und/oder einen Aufguss selbsttätig auszulösen und/oder selbsttätig zu steuern. Der Ofen ist vorzugsweise als Saunaofen ausgebildet. Die Steuerungseinrichtung ist vorzugsweise dazu eingerichtet, abhängig von dem bereitgestellten Steuerungsbefehl die Ofentemperatur und/oder die Heizleistung des Ofens einzustellen. Dadurch, dass die Steuerungseinrichtung dazu eingerichtet ist, die Ofentemperatur und/oder die Heizleistung des Ofens einzustellen, kann die Temperatur innerhalb der Wärmekabine angepasst werden, wobei das Anpassen der Temperatur innerhalb der Wärmekabine das Erhöhen und/oder das Verringern der Temperatur innerhalb der Wärmekabine umfassen kann. Der Ofen kann beispielsweise ein Holzofen, ein Gasofen, ein Elektroofen oder eine Kombination aus den zuvor genannten Öfen sein. Die Steuerungseinrichtung kann dazu eingerichtet sein, das Begießen von heißen Steinen des Ofens mit einer Aufgussflüssigkeit auszulösen. Die Aufgussflüssigkeit kann beispielsweise Wasser sein, wobei das Wasser mit unterschiedlichen Ölen, Aromen und/oder Mineralien versetzt sein kann. Die Steuerungseinrichtung kann dazu eingerichtet sein, abhängig von dem bereitgestellten Steuerungsbefehl die Strahlungsintensität, die Position und/oder die Ausrichtung des einen oder der mehreren Infrarotstrahler selbsttätig einzustellen. Die Steuerungseinrichtung kann dazu eingerichtet sein, abhängig von dem bereitgestellten Steuerungsbefehl die Strömungsgeschwindigkeit und/oder die Strömungsrichtung der von dem einen oder den mehreren Lüftern beschleunigten Luft, und/oder die Position und/oder die Ausrichtung des einen oder der mehreren Lüfter selbsttätig einzustellen. Der eine oder die mehreren Lüfter sind vorzugsweise an dem Möbel angeordnet und/oder befestigt. Das Möbel kann fest in der Kabine integriert oder als portables Möbel ausgebildet sein kann.

Außerdem ist eine Weiterbildung der erfindungsgemäßen Wärmekabineneinrichtung vorteilhaft, bei welcher das Möbel der Wärmekabine als Liege ausgebildet und die Steuerungseinrichtung dazu eingerichtet ist, abhängig von dem bereitgestellten Steuerungsbefehl die Position, insbesondere die Höhe, der Liegefläche der Liege und/oder die Neigung von einer oder mehreren Abschnitten der Liegefläche der Liege einzustellen. Die Liege umfasst vorzugsweise einen oder mehrere elektrisch ansteuerbare Aktuatoren, wie beispielsweise einen oder mehrere Elektromotoren, mit welchen der Liegezustand veränderbar ist. Beispielweise sind der eine oder die mehreren elektrisch ansteuerbaren Aktuatoren dazu eingerichtet, die Liege oder einen Teil der Liege anzuheben, abzusenken, zu verschwenken und/oder zu drehen. Dadurch, dass die Steuerungseinrichtung dazu eingerichtet ist, abhängig von dem bereitgestellten Steuerungsbefehl die Höhe der Liegefläche der Liege einzustellen, kann die sich auf der Liege befindliche Person in eine andere Temperaturzone verfahren werden. Darüber hinaus kann die Steuerungseinrichtung dazu eingerichtet sein, abhängig von dem bereitgestellten Steuerungsbefehl die Liege derart zu verfahren, dass die Entfernung der Liege zu einem Ofen oder einem oder mehreren Infrarotstrahlern der Wärmekabine eingestellt wird. Die Steuerungseinrichtung kann dazu eingerichtet sein, abhängig von dem bereitgestellten Steuerungsbefehl die Neigung der Rückenlehne der Liege und/oder die Neigung des Bein- beziehungsweise Wadenablageabschnitts der Liege selbsttätig zu ändern.

In einer besonders bevorzugten Ausführungsform der erfindungsgemäßen Wärmekabineneinrichtung sind der eine oder die mehreren Sensoren dazu eingerichtet, die Temperatur von einer oder mehreren Körperregionen einer Person, die Herzfrequenz einer Person, den Blutdruck einer Person und/oder die Atemfrequenz einer Person während der Nutzung der Wärmekabine zu erfassen. Ein oder mehrere Sensoren können zum Erfassen der Temperatur von einer oder mehreren Körperregionen der Person als Temperatursensoren ausgebildet sein, welche dazu eingerichtet sind, Wärmestrahlung zu erfassen. Der eine oder die mehreren Sensoren können dazu eingerichtet sein, die Temperatur von einer oder mehreren Körperregionen der Person während der Nutzung der Wärmekabine berührungslos und/oder durch eine Berührung der entsprechenden Körperregion zu erfassen. Ein oder mehrere Sensoren können zum Erfassen der Atemfrequenz der Person dazu eingerichtet sein, Brustumfangsveränderungen der die Wärmekabine nutzenden Person zu Erfassen, wobei die Brustumfangsveränderungen mittels eines Brustgurts oder durch optische Sensoren erfasst werden können. Der eine oder die mehreren Sensoren können dazu eingerichtet sein, die Vitalparameter der Person während der Nutzung der Wärmekabine in regelmäßigen zeitlichen Abständen zu erfassen, wie beispielsweise jede Sekunde oder jede 5 Sekunden, wobei die Erfassungsfrequenz der verschiedenen Vitalparameter unterschiedlich sein kann.

Vorzugsweise ist ein oder sind mehrere Sensoren der erfindungsgemäßen Wärmekabineneinrichtung in einem portablen elektronischen Gerät, insbesondere in einem Armband, einem Brustgurt, einem Ohreinsatz, einem Fingeraufsatz, einer Fingerklemme und/oder einem anderen am Körper tragbaren Gerät, integriert. Der Ohreinsatz kann beispielsweise als Ohrstöpsel und/oder zur Erfassung der Herzfrequenz, der Körpertemperatur und/oder der Sauerstoffsättigung des Blutes ausgebildet sein. Die Wärmekabineneinrichtung kann auch mehrere portable elektronische Geräte aufweisen. Vorzugsweise ist das eine oder sind die mehreren portablen elektronischen Geräte signalleitend mit der Steuerungseinrichtung und/oder mit dem mobilen Endgerät verbunden. Alternativ oder zusätzlich sind ein oder mehrere Sensoren als stationäre Sensoren der Wärmekabine ausgebildet. Die stationären Sensoren der Wärmekabine können beispielsweise in einem Möbel der Wärmekabine, wie in einem Handgriff, integriert sein.

Die Datenbank befindet sich vorzugsweise auf einem entfernt von der Wärmekabine positionierten Zentralrechner, wobei die Wärmekabine vorzugsweise ein Kommunikationsmodul umfasst, welches dazu eingerichtet ist, die erfassten Vitalparameter an den Zentralrechner, insbesondere unter Verwendung des Internets, zu senden. Außerdem kann die Wärmekabineneinrichtung eine Empfängereinrichtung aufweisen, welche dazu eingerichtet ist, die erfassten Vitalparameter von dem einen oder den mehreren portablen elektronischen Geräten zu empfangen, insbesondere über Bluetooth oder Nahfeldkommunikation. Außerdem kann die Empfängereinrichtung dazu eingerichtet sein, die erfassten Vitalparameter an die Steuerungseinrichtung der Wärmekabine, das mobile Endgerät und/oder an den Zentralrechner zu senden. Wobei die Steuerungseinrichtung und/oder das mobile Endgerät dazu eingerichtet sein können, die erfassten Vitalparameter an den Zentralrechner zu senden. Die Auswerteeinrichtung der Wärmekabineneinrichtung kann beispielsweise Bestandteil des Zentralrechners oder der Steuerungseinrichtung der Wärmekabine sein. Die Datenbank ist dazu eingerichtet, die erfassten Vitalparameter von mehreren zeitlich beabstandeten Nutzungen der Wärmekabine durch die Person zu speichern. Somit kann die Datenbank als selbstlernend verstanden werden. Die aus den in der Datenbank gespeicherten Vitalparametern abgeleiteten Handlungsempfehlungen werden auf diese Weise mit der Zeit immer präziser. Ferner werden die aus den in der Datenbank gespeicherten Vitalparametern abgeleiteten Steuerungsbefehle für die Wärmekabine auf diese Weise mit der Zeit immer präziser.

In einer weiteren Ausführungsform verfügt die erfindungsgemäße Wärmekabineneinrichtung über eine Alarm- und/oder Notruf-Funktion. Vorzugsweise ist die Auswerteeinrichtung dazu eingerichtet, die aktuellen und/oder die in der Datenbank gespeicherten Vitalparameter zum Erfassen eines bedrohlichen Gesundheitszustands auszuwerten und bei Erfassung eines solchen Gesundheitszustands einen Alarm auszulösen und/oder einen Notruf an eine Rettungszentrale abzusenden. Das frühzeitige Erfassen eines bedrohlichen und gegebenenfalls lebensgefährlichen Gesundheitszustandes vor, während oder nach der Nutzung der Wärmekabine und das anschließende Auslösen eines Alarms oder das Absenden eines Notrufs kann irreversible gesundheitliche Schäden vermeiden oder sogar eine lebensrettende Maßnahme sein. Ferner kann die Wärmekabineneinrichtung über eine Warnfunktion und/oder über eine Notabschaltungsfunktion verfügen. Vorzugsweise werden bei der Warnfunktion und/oder der Notabschaltungsfunktion die aktuellen und/oder die in der Datenbank gespeicherten Vitalparameter zum Erfassen eines bedrohlichen Gesundheitszustands ausgewertet und bei Erfassung eines solchen Gesundheitszustands eine Warnung ausgegeben und/oder eine Notabschaltung der Wärmekabine ausgeführt.

In einer besonders bevorzugten Ausführungsform weist die erfindungsgemäße Wärmekabineneinrichtung ein portables elektronisches Gerät, wie ein Armband, einen Brustgurt, einen Ohreinsatz, einen Fingeraufsatz, eine Fingerklemme und/oder ein anderes am Körper tragbares Gerät auf, welches Sensoren zum Erfassen von Vitalparametern einer Person aufweist. Das elektronische Gerät ist dazu eingerichtet, die erfassten Vitalparameter über Bluetooth an eine als Bluetooth-Empfänger ausgebildete Empfängereinrichtung zu senden. Die Empfängereinrichtung ist dazu eingerichtet, die Vitalparameter an die Steuerungseinrichtung der Wärmekabine weiterzuleiten, wobei die Steuerungseinrichtung einen lokalen Netzwerkanschluss aufweist. Ferner weist die Steuerungseinrichtung ein Kommunikationsmodul auf, mittels welchem eine Internetverbindung herstellbar ist. Die Wärmekabineneinrichtung weist ferner einen als Server ausgebildeten Zentralrechner mit einer Datenbank und ein als Smartphone ausgebildetes mobiles Endgerät auf. Bei dieser Konfiguration kann das portable elektronische Gerät zuerst die Vitalparameter einer Person während der Nutzung der Wärmekabine erfassen. Diese werden dann über Bluetooth an die Empfängereinrichtung und dann von der Empfängereinrichtung über ein lokales Netzwerk an die Steuerungseinrichtung der Wärmekabine gesendet. Die Steuerungseinrichtung der Wärmekabine sendet die erfassten Vitalparameter dann an den Zentralrechner. Der Zentralrechner speichert die Daten auf einer Datenbank und erzeugt durch eine Auswertung der Daten Handlungsempfehlungen für die Person und/oder Steuerungsbefehle für die Wärmekabine. Der Zentralrechner sendet die Handlungsempfehlungen und/oder die Steuerungsbefehle dann über das Internet zurück an die Steuerungseinrichtung, sodass die Steuerungseinrichtung beispielsweise die Anpassung der Temperatur innerhalb der Wärmekabine und/oder Liegeposition der Person initiieren kann. Ferner können die Handlungsempfehlungen über eine kabineneigene Wiedergabeeinrichtung der Person bereitgestellt werden. Der Zentralrechner kann die Handlungsempfehlungen auch über das Internet an das mobile Endgerät senden, welches der Person die Handlungsempfehlung über eine entsprechende Anwendung bereitstellen kann. Bevor genügend Vitalparameter in einer Datenbank vorliegen um Handlungsempfehlungen und/oder Steuerungsbefehle über das Internet an die Steuerungseinrichtung zu senden, sodass die Steuerungseinrichtung beispielsweise die Anpassung der Temperatur innerhalb der Wärmekabine und/oder Liegeposition der Person initiieren kann, werden zu Beginn Handlungsempfehlungen und/oder Steuerungsbefehle über das Internet an die Steuerungseinrichtung gesendet, die auf Annahmen basieren.

In einer weiteren bevorzugten Ausführungsform weist die erfindungsgemäße Wärmekabineneinrichtung ein portables elektronisches Gerät, wie ein Armband, einen Brustgurt, einen Ohreinsatz, einen Fingeraufsatz, eine Fingerklemme oder ein anderes am Körper tragbares Gerät, auf, welches Sensoren zum Erfassen von Vitalparametern einer Person aufweist. Das elektronische Gerät ist dazu eingerichtet, die erfassten Vitalparameter über Bluetooth an eine als Bluetooth-Empfänger ausgebildete Empfängereinrichtung zu senden. Die Empfängereinrichtung ist dazu eingerichtet, die Vitalparameter über das Internet an einen als Server ausgebildeten Zentralrechner zu senden. Ferner umfasst die Wärmekabineneinrichtung ein als Smartphone ausgebildetes mobiles Endgerät. Bei dieser Konfiguration kann das portable elektronische Gerät zuerst die Vitalparameter einer Person während der Nutzung der Wärmekabine erfassen. Diese werden dann über Bluetooth an die Empfängereinrichtung und von der Empfängereinrichtung über das Internet an den Zentralrechner gesendet. Der Zentralrechner speichert die Daten auf einer Datenbank und erzeugt durch eine Auswertung der Daten eine Handlungsempfehlung für die Person. Die Handlungsempfehlung wird über das Internet an das mobile Endgerät gesendet, welches der Person die Handlungsempfehlung über eine entsprechende Anwendung bereitstellen kann.

In einer anderen bevorzugten Ausführungsform weist die erfindungsgemäße Wärmekabineneinrichtung ein portables elektronisches Gerät, wie ein Armband, einen Brustgurt, einen Ohreinsatz, einen Fingeraufsatz, eine Fingerklemme oder ein anderes am Körper tragbares Gerät, auf, welches Sensoren zum Erfassen von Vitalparametern einer Person aufweist. Das elektronische Gerät ist dazu eingerichtet, die erfassten Vitalparameter über Bluetooth an ein als Smartphone ausgebildetes mobiles Endgerät zu senden. Ferner umfasst die Wärmekabineneinrichtung einen Zentralrechner. Bei dieser Konfiguration kann das portable elektronische Gerät zuerst die Vitalparameter einer Person während der Nutzung der Wärmekabine erfassen. Diese werden dann über Bluetooth an das mobile Endgerät und von dem mobilen Endgerät über das Internet an den Zentralrechner gesendet. Der Zentralrechner speichert die Daten auf einer Datenbank und erzeugt durch eine Auswertung der Daten eine Handlungsempfehlung für die Person. Die Handlungsempfehlung wird über das Internet an das mobile Endgerät gesendet, welches der Person die Handlungsempfehlung über eine entsprechende Anwendung bereitstellen kann.

In einer weiteren bevorzugten Ausführungsform der erfindungsgemäßen Wärmekabineneinrichtung ist die Auswerteeinrichtung dazu eingerichtet, auf Basis von Annahmen eine Handlungsempfehlung für die Nutzung der Wärmekabine zu erzeugen und bereitzustellen. Dies kann insbesondere dann vorteilhaft sein, wenn nicht ausreichend Vitalparameter zur Erzeugung einer Handlungsempfehlung bereitstehen und/oder die Übertragung der Vitalparameter, beispielsweise aufgrund einer fehlenden Internetverbindung, unterbrochen ist.

Nachfolgend werden bevorzugte Ausführungsformen der Erfindung unter Bezugnahme auf die beiliegenden Zeichnungen näher erläutert und beschrieben. Dabei zeigen:
- Fig. 1: ein Ausführungsbeispiel der erfindungsgemäßen Wärmekabineneinrichtung in einer schematischen Darstellung; und
- Fig. 2: ein Ausführungsbeispiel des erfindungsgemäßen Verfahrens als Blockdiagramm.

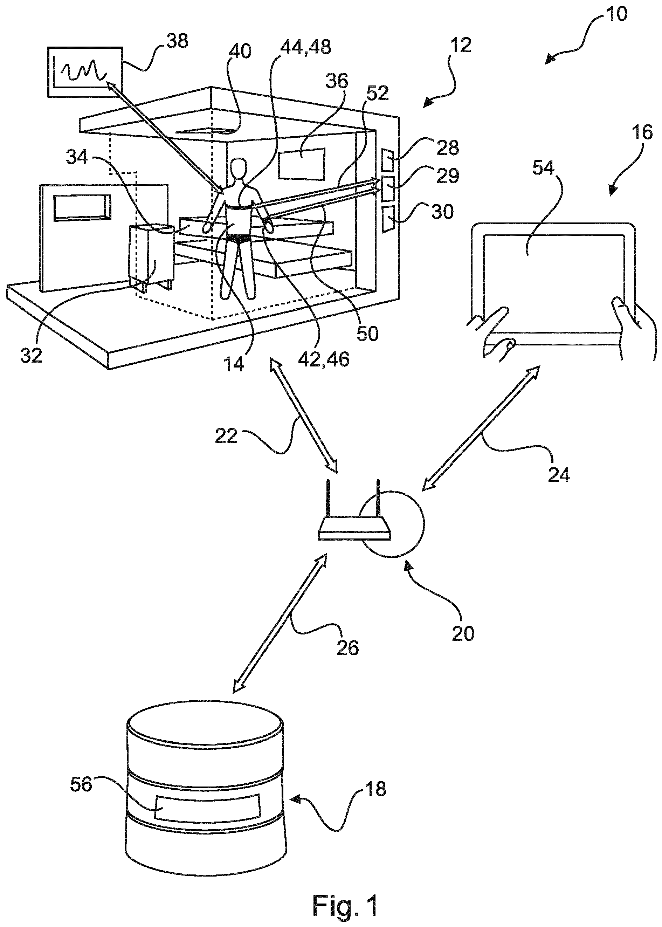

Gemäß Fig. 1 umfasst die Wärmekabineneinrichtung 10 eine Wärmekabine 12, welche von einer Person 14 nutzbar ist, ein mobiles Endgerät 16 und einen Zentralrechner 18.

Die Wärmekabine 12, das mobile Endgerät 16 und der Zentralrechner 18 sind jeweils über die Kommunikationsverbindungen 22-26 mit dem Internet 20 verbunden, sodass ein Datenaustausch zwischen der Wärmekabine 12, dem mobilen Endgerät 16 und dem Zentralrechner 18 erfolgen kann.

Die Wärmekabine 12 ist als Sauna ausgebildet und weist eine Steuerungseinrichtung 28 auf, welche dazu eingerichtet ist, abhängig von einem bereitgestellten Steuerungsbefehl den Betrieb der Wärmekabine 12 zu steuern. Die Steuerungseinrichtung 28 ist signalleitend mit einer Bedieneinheit 30, einem Ofen 32 und einem als einstellbare Saunaliege ausgebildeten Möbel 34 verbunden. Die Bedieneinheit 30 weist eine Anzeige zur Wiedergabe von Informationen und Druckknöpfe zur manuellen Eingabe von Steuerbefehlen auf. Alternativ oder zusätzlich kann die Bedieneinheit 30 auch einen Touchscreen aufweisen. Über die Bedieneinheit 30 können Betriebsparameter der Wärmekabine 12, wie beispielsweise die Saunatemperatur, manuell eingestellt werden. Der Ofen 32 ist als Elektro-Saunaofen ausgebildet. Das als einstellbare Saunaliege ausgebildete Möbel 34 weist mehrere Aktuatoren auf, mittels welchen die Höhe der Liegefläche einstellbar ist. Außerdem können an der Saunaliege mehrere Lüfter angeordnet sein, welche dazu eingerichtet sind, eine Luftströmung in dem Bereich der Liegefläche der Saunaliege zu erzeugen.

Die Steuerungseinrichtung 28 ist dazu eingerichtet, abhängig von dem bereitgestellten Steuerungsbefehl den Ofen 32, das Möbel 34 und die mehreren Lüfter selbsttätig einzustellen.

Die Wärmekabine 12 umfasst ferner eine Wiedergabeeinrichtung 36, welche einen Display und mehrere Lautsprecher umfasst und dazu eingerichtet ist, der die Wärmekabine 12 nutzenden Person 14 Informationen bereitzustellen. Die von der Wiedergabeeinrichtung 36 bereitgestellten Informationen können beispielsweise Betriebsparameter der Wärmekabine 36 und/oder Handlungsempfehlungen für die Person umfassen oder der Unterhaltung der Person 14 dienen.

Die Person 14 trägt ein Armband 46 und einen Brustgurt 48. Das Armband 46 und der Brustgurt 48 umfassen jeweils Sensoren 42, 44, welche dazu eingerichtet sind, Vitalparameter 38 der Person 14 zu erfassen. Der Sensor 42 ist dazu eingerichtet, die Herzfrequenz der Person 14 zu erfassen. Der Sensor 44 ist dazu eingerichtet, die Atemfrequenz der Person 14 zu erfassen. In anderen Ausführungsformen können auch mehrere Sensoren 42, 44 in dem Armband oder dem Brustgurt integriert sein, sodass die Person auf das Tragen des jeweils anderen portablen elektronischen Geräts verzichten kann. Die Wärmekabine 12 weist außerdem einen stationären Sensor 40 auf, welcher dazu eingerichtet ist, die Körpertemperatur der Person 14 zu erfassen. Alternativ oder zusätzlich können auch Sensoren des Armbands und/oder des Brustgurts die Körpertemperatur der Person 14 erfassen. Die Sensoren 40-44 stellen die erfassten Vitalparameter 38 einer Empfängereinrichtung 29 bereit, welche als Bluetooth-Empfänger ausgebildet ist. Hierzu verfügen das Armband 46 und der Brustgurt 48 jeweils über ein Kommunikationsmodul. Der Datenaustausch zwischen dem Kommunikationsmodul des Armbands 46 und der Empfängereinrichtung 29 erfolgt über die Bluetooth-Verbindung 50. Der Datenaustausch zwischen dem Kommunikationsmodul des Brustgurts 48 und der Empfängereinrichtung 29 erfolgt über die Bluetooth-Verbindung 52. Die Empfängereinrichtung 29 ist signalleitend über eine lokale Netzwerkverbindung mit einem Kommunikationsmodul der Steuerungseinrichtung 28 verbunden, sodass die Empfängereinrichtung 29 die erfassten Vitalparameter 38 dem Kommunikationsmodul der Steuerungseinrichtung 28 bereitstellen kann. Das Kommunikationsmodul der Steuerungseinrichtung 28 ist dazu eingerichtet, die erfassten Vitalparameter 38 über das Internet 20 an den Zentralrechner 18 zu senden. Der Zentralrechner 18 ist entfernt von der Wärmekabine 12 positioniert und umfasst eine Datenbank 56, welche dazu eingerichtet ist, die erfassten Vitalparameter 38 zu speichern. Der Zentralrechner 56 weist außerdem eine Auswerteeinrichtung auf, welche dazu eingerichtet ist, die erfassten Vitalparameter 38 der Person 14 abzurufen und auszuwerten.

Die Auswerteeinrichtung kann in anderen Ausführungsbeispielen auch in das mobile Endgerät 16 oder in die Steuerungseinrichtung 28 der Wärmekabine 12 integriert sein. Die Auswertung der in der Datenbank 56 gespeicherten Vitalparameter 38 der Person 14 erfolgt derart, dass eine personenbezogene Handlungsempfehlung für die Nutzung der Wärmekabine 12 erzeugt wird. Diese Handlungsempfehlung kann der Person beispielsweise über die als Touchscreen ausgebildete Wiedergabeeinrichtung 54 des mobilen Endgeräts 16 zur Verfügung gestellt werden. Hierzu ist auf dem mobilen Endgerät eine entsprechende Anwendung installiert, über welche auch der direkte Zugriff auf die Daten der Person 14, welche in der Datenbank 56 auf dem Zentralrechner 18 gespeichert sind, erfolgen kann. Alternativ oder zusätzlich kann der Person 14 auch während der Nutzung der Wärmekabine 12 eine Handlungsempfehlung über die Wiedergabeeinrichtung 36 bereitgestellt werden. Hierzu wird die von der Auswerteeinrichtung des Zentralrechners 18 erzeugte Handlungsempfehlung von dem Zentralrechner 18 über das Internet 20 an das Kommunikationsmodul der Steuerungseinrichtung 28 gesendet, sodass die Steuerungseinrichtung 28 die Handlungsempfehlung zur Wiedergabe an die Wiedergabeeinrichtung 36 weiterleiten kann. Außerdem ist es möglich im Falle einer störenden und/oder ausfallenden Verbindung zu dem Internet 20, die erfassten Vitalparameter 38 auf dem Armband 46 zwischen zu speichern und der Datenbank 56 zu einem späteren Zeitpunkt zu übermitteln. Währenddessen werden von der Steuerungseinrichtung 28 voreingestellte Handlungsempfehlungen zur Wiedergabe an die Wiedergabeeinrichtung 36 weitergeleitet.

Die in dem Zentralrechner 18 integrierte Auswerteeinrichtung ist außerdem dazu eingerichtet, auf Basis der in der Datenbank 56 gespeicherten Vitalparameter 38 einen Steuerungsbefehl für die Wärmekabine 12 zu erzeugen und der Steuerungseinrichtung 28 der Wärmekabine 12 über das Internet 20 bereitzustellen, sodass abhängig von dem bereitgestellten Steuerungsbefehl der Ofen 32, das Möbel 34 und die mehreren Lüfter selbsttätig eingestellt werden.

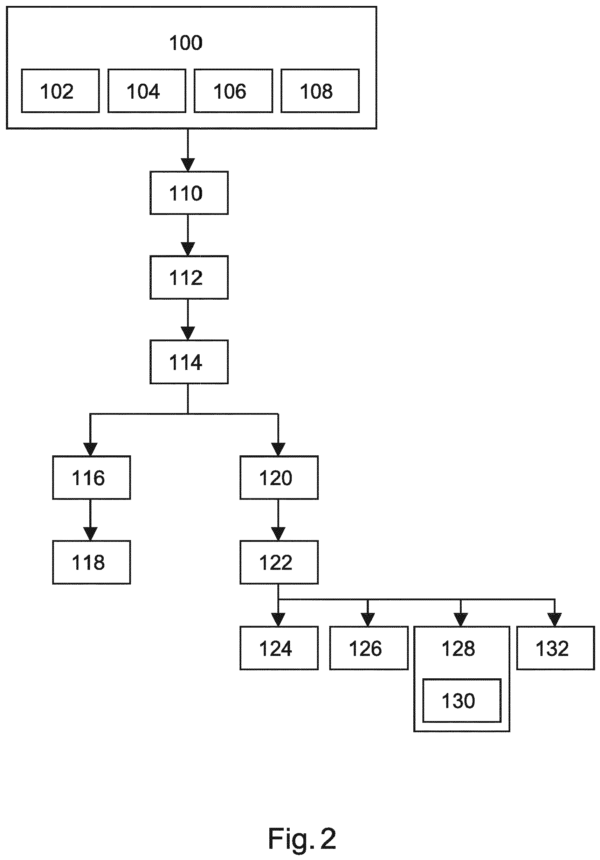

Gemäß Fig. 2 wird das Verfahren für die Nutzung einer Wärmekabine 12 mit dem folgenden Schritt eingeleitet:
100) Erfassen von Vitalparametern 38 einer Person 14.

Das Erfassen der Vitalparameter 38 der Person 14 erfolgt während der Nutzung der Wärmekabine 12 und umfasst die folgenden vier Schritte:
102) Erfassen der Temperatur von mehreren Körperregionen der Person 14 während der Nutzung der Wärmekabine 12,
104) Erfassen der Herzfrequenz der Person 14 während der Nutzung der Wärmekabine 12,
106) Erfassen des Blutdrucks der Person 14 während der Nutzung der Wärmekabine 12, und
108) Erfassen der Atemfrequenz der Person 14 während der Nutzung der Wärmekabine 12.

Die Erfassung der Temperatur von mehreren Körperregionen, der Herzfrequenz, des Blutdrucks und der Atemfrequenz erfolgt in regelmäßigen zeitlichen Abständen. Nach der Erfassung der Vitalparameter wird der folgende Schritt ausgeführt:
110) Senden der erfassten Vitalparameter 38 an einen Zentralrechner 18.

Das Senden der erfassten Vitalparameter 38 an einen Zentralrechner 18 erfolgt unter Verwendung des Internets 20. Das Übermitteln der erfassten Vitalparameter 38 an den Zentralrechner 18 erfolgt ebenfalls in regelmäßigen zeitlichen Abständen. Der Zentralrechner 18 ist entfernt von der Wärmekabine 12 positioniert und umfasst eine Datenbank 56. Nachdem die erfassten Vitalparameter 38 an den Zentralrechner 18 gesendet wurden, kann somit der folgende Schritt ausgeführt werden:
112) Speichern der erfassten Vitalparameter 38 in der Datenbank 56.

Die Vitalparameter 38 werden in der Datenbank dem Benutzerprofil der Person 14 zugeordnet, wobei das Benutzerprofil ein anonymisiertes Benutzerprofil ist. Den erfassten Vitalparametern 38 werden in der Datenbank außerdem das Erfassungsdatum und die Erfassungsuhrzeit zugeordnet. Nach dem Speichern der erfassten Vitalparameter 38 in der Datenbank 56 wird dann der folgende Schritt ausgeführt:
114) Bereitstellen der Datenbank 56 zum Auswerten der gespeicherten Vitalparameter 38.

Nachdem die Datenbank 56 zum Auswerten der gespeicherten Vitalparameter 38 bereitgestellt wurde, werden die folgenden zwei Schritte ausgeführt:
116) Auswerten der in der Datenbank 56 gespeicherten Vitalparameter 38 zum Erzeugen einer personenbezogenen Handlungsempfehlung für die Nutzung der Wärmekabine 12, und
118) Bereitstellen der erzeugten personenbezogenen Handlungsempfehlung für die Person 14 mittels einer Anwendung auf einem mobilen Endgerät 16.

Neben der Erzeugung von personenbezogenen Handlungsempfehlungen werden die in der Datenbank 56 gespeicherten Vitalparameter 38 außerdem zur Steuerung der Wärmekabine verwendet. Hierzu werden die folgenden Schritte ausgeführt:
120) Auswerten der in der Datenbank 56 gespeicherten Vitalparameter 38 zum Erzeugen eines Steuerungsbefehls für die Wärmekabine 12, und
122) Bereitstellen des erzeugten Steuerungsbefehls für eine Steuerungseinrichtung 28 der Wärmekabine 12.

Auf Grundlage des erzeugten Steuerungsbefehls werden dann beispielsweise die folgenden Schritte ausgeführt:
124) selbsttätiges Einstellen eines Ofens 32 der Wärmekabine 12 abhängig von dem bereitgestellten Steuerungsbefehl, und
126) selbsttätiges Auslösen eines Aufgusses abhängig von dem bereitgestellten Steuerungsbefehl.

Das selbsttätige Einstellen des Ofens 32 erfolgt zur Anpassung der Temperatur innerhalb der Wärmekabine 12. Das selbsttätige Auslösen des Aufgusses erfolgt zur Anpassung der Luftfeuchtigkeit innerhalb der Wärmekabine 12.

Auf Grundlage des erzeugten Steuerungsbefehls kann beispielsweise auch der folgende Schritt ausgeführt werden:
128) selbsttätiges Einstellen eines Möbels 34 der Wärmekabine 12 abhängig von dem bereitgestellten Steuerungsbefehl.

Das Möbel 34 der Wärmekabine 12 ist als Liege ausgebildet ist, wobei das selbsttätige Einstellen des Möbels 34 der Wärmekabine 12 abhängig von dem bereitgestellten Steuerungsbefehl den folgenden Schritt umfasst:
130) Einstellen der Höhe der Liegefläche der Liege.

An der Liege können mehrere Lüfter angeordnet sein, welche dazu eingerichtet sind, eine Luftströmung in dem Bereich der Liegefläche der Liege zu verursachen, sodass auf Grundlage des erzeugten Steuerungsbefehls auch der folgende Schritt ausgeführt werden kann:
132) selbsttätiges Einstellen der mehreren Lüfter der Wärmekabine 12 abhängig von dem bereitgestellten Steuerungsbefehl.

### Bezugszeichen

- 10: Wärmekabineneinrichtung
- 12: Wärmekabine
- 14: Person
- 16: mobiles Endgerät
- 18: Zentralrechner
- 20: Internet
- 22-26: Kommunikationsverbindungen
- 28: Steuerungseinrichtung
- 29: Empfängereinrichtung
- 30: Bedieneinheit
- 32: Ofen
- 34: Möbel
- 36: Wiedergabeeinrichtung
- 38: Vitalparameter
- 40-44: Sensoren
- 46: Armband
- 48: Brustgurt
- 50, 52: Kommunikationsverbindungen
- 54: Wiedergabeeinrichtung
- 56: Datenbank

- 100-132: Verfahrensschritte

## Patentansprüche

1. Verfahren für die Nutzung einer Wärmekabine (12), mit dem Schritt:
- Erfassen von Vitalparametern (38) einer Person (14), insbesondere während der Nutzung der Wärmekabine (12);
**gekennzeichnet durch** die Schritte:
- Speichern der erfassten Vitalparameter (38) in einer Datenbank (56); und
- Bereitstellen der Datenbank (56) zum Auswerten der gespeicherten Vitalparameter (38).

2. Verfahren nach Anspruch 1,
**gekennzeichnet durch** zumindest einen der folgenden Schritte:
- Auswerten der in der Datenbank (56) gespeicherten Vitalparameter (38) zum Erzeugen einer Handlungsempfehlung für die Nutzung der Wärmekabine (12);
- Bereitstellen der erzeugten Handlungsempfehlung für die Person (14), vorzugsweise mittels einer Anwendung auf einem mobilen Endgerät (16) und/oder einer Wiedergabeeinrichtung (36) der Wärmekabine (12).

3. Verfahren Anspruch 1 oder 2,
**gekennzeichnet durch** zumindest einen der folgenden Schritte:
- Auswerten der in der Datenbank (56) gespeicherten Vitalparameter (38) zum Erzeugen eines Steuerungsbefehls für die Wärmekabine (12);
- Bereitstellen des erzeugten Steuerungsbefehls für eine Steuerungseinrichtung (28) der Wärmekabine (12).

4. Verfahren nach Anspruch 3,
**gekennzeichnet durch** zumindest einen der folgenden Schritte:
- selbsttätiges Einstellen eines Ofens (32) der Wärmekabine (12) abhängig von dem bereitgestellten Steuerungsbefehl;
- selbsttätiges Auslösen und/oder Steuern eines Aufgusses abhängig von dem bereitgestellten Steuerungsbefehl;
- selbsttätiges Einstellen eines oder mehrerer Infrarotstrahler der Wärmekabine (12) abhängig von dem bereitgestellten Steuerungsbefehl;
- selbsttätiges Einstellen eines Möbels (34) der Wärmekabine (12) abhängig von dem bereitgestellten Steuerungsbefehl;
- selbsttätiges Einstellen eines oder mehrerer Lüfter der Wärmekabine (12) abhängig von dem bereitgestellten Steuerungsbefehl.

5. Verfahren nach Anspruch 4,
**dadurch gekennzeichnet, dass** das Möbel (34) der Wärmekabine (12) als Liege ausgebildet ist und das selbsttätige Einstellen des Möbels (34) der Wärmekabine (12) abhängig von dem bereitgestellten Steuerungsbefehl zumindest einen der folgenden Schritte umfasst:
- Einstellen der Position, insbesondere der Höhe, der Liegefläche der Liege;
- Einstellen der Neigung von einem oder mehreren Abschnitten der Liegefläche der Liege.

6. Verfahren nach einem der vorstehenden Ansprüche,
**dadurch gekennzeichnet, dass** das Erfassen von Vitalparametern (38) der Person (14) während der Nutzung der Wärmekabine (12) einen, mehrere oder sämtliche der folgenden Schritte umfasst:
- Erfassen der Temperatur von einer oder mehreren Körperregionen der Person (14) während der Nutzung der Wärmekabine (12);
- Erfassen der Herzfrequenz der Person (14) während der Nutzung der Wärmekabine (12);
- Erfassen des Blutdrucks der Person (14) während der Nutzung der Wärmekabine (12);
- Erfassen der Atemfrequenz der Person (14) während der Nutzung der Wärmekabine (12).

7. Verfahren nach einem der vorstehenden Ansprüche,
**dadurch gekennzeichnet, dass** sich die Datenbank (56) auf einem entfernt von der Wärmekabine (12) positionierten Zentralrechner (18) befindet und das Verfahren den folgenden Schritt umfasst:
- Senden der erfassten Vitalparameter (38) an den Zentralrechner (18), insbesondere unter Verwendung des Internets (20).

8. Wärmekabineneinrichtung (10), mit
- einer Wärmekabine (12); und
- einem oder mehreren Sensoren (40-44), welche jeweils dazu eingerichtet sind, Vitalparameter (38) einer Person (14), insbesondere während der Nutzung der Wärmekabine (12), zu erfassen;
**gekennzeichnet durch**
- eine Datenbank (56), welche dazu eingerichtet ist, die erfassten Vitalparameter (38) zu speichern; und
- eine Auswerteeinrichtung, welche dazu eingerichtet ist, die in der Datenbank (56) gespeicherten Vitalparameter (38) auszuwerten.

9. Wärmekabineneinrichtung (10) nach Anspruch 8,
**dadurch gekennzeichnet, dass** die Auswerteeinrichtung dazu eingerichtet ist, auf Basis der in der Datenbank (56) gespeicherten Vitalparameter (38) eine Handlungsempfehlung für die Nutzung der Wärmekabine (12) zu erzeugen und bereitzustellen.

10. Wärmekabineneinrichtung (10) nach Anspruch 9,
**gekennzeichnet durch** ein mobiles Endgerät (16), welches dazu eingerichtet ist, der Person (14) die Handlungsempfehlung bereitzustellen, insbesondere mittels einer auf dem mobilen Endgerät (16) installierten Anwendung und/oder einer Wiedergabeeinrichtung (36) der Wärmekabine (12).

11. Wärmekabineneinrichtung (10) nach einem der Ansprüche 8 bis 10,
**dadurch gekennzeichnet, dass** die Auswerteeinrichtung dazu eingerichtet ist, auf Basis der in der Datenbank (56) gespeicherten Vitalparameter (38) einen Steuerungsbefehl für die Wärmekabine (12) zu erzeugen und bereitzustellen.

12. Wärmekabineneinrichtung (10) nach Anspruch 11,
**gekennzeichnet durch** eine Steuerungseinrichtung (28), welche dazu eingerichtet ist, abhängig von dem bereitgestellten Steuerungsbefehl einen Ofen (32), einen oder mehrere Infrarotstrahler, ein Möbel (34) und/oder einen oder mehrere Lüfter der Wärmekabine (12) selbsttätig einzustellen und/oder einen Aufguss selbsttätig auszulösen und/oder selbsttätig zu steuern.

13. Wärmekabineneinrichtung (10) nach Anspruch 12,
**dadurch gekennzeichnet, dass** das Möbel (34) der Wärmekabine (12) als Liege ausgebildet und die Steuerungseinrichtung (28) dazu eingerichtet ist, abhängig von dem bereitgestellten Steuerungsbefehl die Position, insbesondere die Höhe, der Liegefläche der Liege und/oder die Neigung von einer oder mehreren Abschnitten der Liegefläche der Liege einzustellen.

14. Wärmekabineneinrichtung (10) nach einem der Ansprüche 8 bis 13,
**dadurch gekennzeichnet, dass** der eine oder die mehreren Sensoren (40-44) dazu eingerichtet sind, die Temperatur von einer oder mehreren Körperregionen einer Person (14), die Herzfrequenz einer Person (14), den Blutdruck einer Person (14) und/oder die Atemfrequenz einer Person (14) während der Nutzung der Wärmekabine (12) zu erfassen.

15. Wärmekabineneinrichtung (10) nach einem der Ansprüche 8 bis 14,
**dadurch gekennzeichnet, dass** ein oder mehrere Sensoren (40-44) in einem portablen elektronischen Gerät, insbesondere in einem Armband (46), einem Brustgurt (48), einem Ohreinsatz, einem Fingeraufsatz und/oder einer Fingerklemme, integriert sind und/oder wobei ein oder mehrere Sensoren (40-44) als stationäre Sensoren (40-44) der Wärmekabine (12) ausgebildet sind.
